# EUROPEAN PATENT APPLICATION

(11) **EP 4 501 324 A1**
(43) Date of publication of application: **05.02.2025**
(21) Application number: 23775061.7
(22) Date of filing: 24.03.2023
(51) Int. Cl.: A61K 31/421, A61P 17/02

(54) **WOUND TREATMENT COMPOSITION**

(30) Priority: 25.03.2022 JP 2022049233
(71) Applicant: Otsuka Pharmaceutical Co., Ltd., Tokyo 101-8535 (JP)
(72) Inventor: NAKASHIMA, Takako, Osaka-shi, Osaka 540-0021 (JP); ARICHIKA, Naoya, Osaka-shi, Osaka 540-0021 (JP); HIYAMA, Hidetaka, Osaka-shi, Osaka 540-0021 (JP); MATSUDA, Takakuni, Osaka-shi, Osaka 540-0021 (JP); MATSUMOTO, Kengo, Osaka-shi, Osaka 540-0021 (JP); YAMASHITA, Daisuke, Osaka-shi, Osaka 540-0021 (JP)
(74) Representative: Hoffmann Eitle
(86) International application number: PCT/JP2023/011678
(87) International publication number: WO 2023/182468

(57) **Abstract**

The present disclosure provides a composition for a treatment for a wound, and the like. N-({2-[4-(difluoromethoxy)-3-(propan-2-yloxy)phenyl]-1,3-oxazol-4-yl}methyl)-2-ethoxybenzamide or its salt is useful as an active ingredient of a composition for a treatment for a wound.

## Description

### Technical Field

The present disclosure relates to a treatment and the like for a wound using N-({2-[4-(difluoromethoxy)-3-(propan-2-yloxy)phenyl]-1,3-oxazol-4-yl}methyl)-2-ethoxybenzamide (hereinafter, sometimes referred to as a compound A) or its salt.

### Background Art

Wounds, specifically, injuries such as pressure ulcers, burns, lacerations, abrasions, erosions, and ulcers are caused by various external factors and endogenous factors in the skin and the mucosa, and in the healing process of these wounds, healing is considered to proceed under a complex relationship of various factors such as inflammation, cell proliferation, cell migration, and growth factors. For promotion of recovery from a skin wound, wound bed preparation has been proposed that includes removal of necrotic tissue, prevention of infection by bacteria and the like, suppression of inflammation, prevention of dryness, and management of exudate. Examples of a treatment for a skin wound include a method in which a wound dressing is used for preventing dryness, and a method in which disinfection or an antibiotic is used for preventing infection. However, it has been reported that problems of resistant bacteria are rapidly increasing due to excessive use or erroneous use of antibiotics, and that cytotoxic disinfectants or antibacterial agents rather inhibit wound healing (Non-patent Literature (NPL) 1). Also in the mucosa, various symptoms such as pain are caused by the wound, and the wound site of the mucosa is at risk for infection with microorganisms, and therefore it is considered that development of a drug that promotes wound healing in the mucosa is significant for life support and for maintenance and improvement of quality of life (QOL) of people. However, at present, an innovative therapeutic agent for promotion of mucosal wound healing has been undeveloped.

The healing process of a chronic skin wound is divided into three phases: inflammatory phase, proliferative phase, and remodeling phase. Different cells, cytokines, and growth factors play a major role in the wound healing depending on the disease stage. Medicines usable for promotion of wound healing are limited, and example of the Medicines used include prostaglandin E1 ointment (PROSTANDIN (trademark) ointment), trafermin (basic fibroblast growth factor, bFGF) preparation (FIBLAST (trademark) spray), and bucladesine sodium ointment (Actosin (trademark) ointment), but these are used in the case of a granulation/epithelium formation phase of a deep chronic skin wound.

Oral mucosal wounds included in mucosal wounds are due to many different causes (such as infectious diseases, systemic diseases, physical or chemical stimulators, and allergic reactions), and causes a plurality of ulcers with pain and oral mucositis. Oral mucositis is one of significant adverse events frequently observed particularly with a treatment for cancer (anticancer agents and radiation). A cancer patient who has developed oral mucositis may suffer from eating disorder, deglutition disorder, and the like accompanying the oral mucositis, resulting in deterioration of the nutritional status. Sonis et al. have proposed a process of onset and healing of oral mucositis associated with cancer chemoradiotherapy, and the process includes five steps of initiation, upregulation and message generation, amplification and signaling, ulceration, and healing, but the mechanism of the steps is very complicated and has not yet been completely elucidated (NPL 2). Palifermin, which is a keratinocyte growth factor (KGF), has been approved in the United States as a therapeutic agent for oral mucositis in a hematopoietic stem cell transplantation patient, but use of this agent is limited, and an effective method of treatment for oral mucositis is desired.

Ocular mucosal wounds as another aspect of mucosal wounds include keratoconjunctival epithelial disorders. A keratoconjunctival epithelial disorder is a symptom caused by damage to the corneal epithelium or the conjunctival epithelium due to, for example, an endogenous disease such as Sjogren's syndrome, Stevens-Johnson syndrome, or sicca symptoms (dry eye) or an exogenous disease caused by surgery, a drug, injury, contact lens wearing, or the like, and strong pain occurs on the surface of the eye. Corneal wounds accompanied by a deep defect reaching the basement membrane under the corneal epithelium are classified into "corneal erosion", and corneal wounds accompanied by a shallow and small defect of the corneal epithelium are classified into "superficial punctate keratopathy". Dry eye is a disease in which the cornea and the conjunctiva are damaged by deterioration of the stability of the tear film due to changes in the amount and the quality of tears caused by various factors. In dry eye, chronic and mild "superficial punctate keratopathy" is repeated that causes eye discomfort and a visual function abnormality, and thus dry eye is often accompanied by a keratoconjunctival epithelial disorder. In Japan, an ophthalmic solution of sodium hyaluronate is clinically used as a therapeutic agent for a keratoconjunctival epithelial disorder, and an ophthalmic solution of diquafosol sodium or an ophthalmic solution of rebamipide is clinically used for dry eye (a patient with a keratoconjunctival epithelial disorder associated with a tear abnormality and diagnosed as dry eye). However, since dry eye is a disease that significantly worsen the quality of life (QOL) of the patient, development of a more effective dry eye therapeutic agent with new action mechanism has been awaited.

Meanwhile, Patent Literature (PTL) 1 to PTL 6 disclose a compound A and its crystal as a compound having a phosphodiesterase 4 (PDE4) inhibitory action, and a production method and a preparation of the compound A. As described in NPL 3, it is considered that the compound A inhibits PDE4 to suppress production of a chemical transmitter such as an inflammatory cytokine, and exhibits an anti-inflammatory action to improve a symptom of atopic dermatitis. However, the above literature has no description of the use of the compound A for a wound treatment.

PTL 7 discloses that a pyridine-substituted naphthalene derivative, a pyridine-substituted isoquinoline derivative, and the like have an action of promoting healing of a skin injury such as a wound. However, PTL 7 has no description of a wound healing effect by a substance other than the above-described derivatives, and describes that the action mechanism of the wound healing effect by the derivatives is unknown. NPL 4 describes that CHF6001, which is a PDE4 inhibitor, suppresses growth of human keratinocytes, and that reepithelialization is decreased in a scratch model after treatment with CHF6001.

### Citation List

### Patent Literature

PTL 1: WO2007/058338
PTL 2: JP2008-308496A
PTL 3: WO2014/034958
PTL 4: WO2017/115780
PTL 5: WO2019/194211
PTL 6: JP2021-59538A
PTL 7: JP2006-151964A

### Non-patent Literature

NPL 1: Negut I, et. al., Molecules 2018, 23, 2392
NPL 2: Sonis ST, J Support Oncol.2004, 2, 3
NPL 3: Moizerto (trademark) ointment 0.3%/1% package insert
NPL 4: Woodby B, et. al., Arch Biochem Biophys.2020, 685, 108355

### Summary of Invention

### Technical Problem

The present inventors have conducted studies for the purpose of, for example, providing a new treatment composition for a wound (such as a pressure ulcer).

### Solution to Problem

The present inventors have found that the compound A exhibits a wound treatment effect in a diabetic mouse wound model. Furthermore, the present inventors have found that the compound A exhibits an epidermis formation promoting action in a scratch assay of normal human epidermal keratinocytes. The present inventors have further conducted studies and found that in a rat oral mucosal wound model and a rat corneal epithelial wound model, the compound A exhibits a healing promoting effect on a mucosal wound (oral mucosal wound, ocular mucosal wound). In addition, the present inventors have found that the compound A exhibits a corneal epithelium formation promoting action in a scratch assay using human corneal epithelial cells.

The present disclosure includes, for example, the subject matter described in the following items.

[1] A composition containing N-({2-[4-(difluoromethoxy)-3-(propan-2-yloxy)phenyl]-1,3-oxazol-4-yl}methyl)-2-ethoxybenzamide or its salt,
the composition for a treatment for a wound.

[2] The composition according to the item [1], wherein the wound is a skin wound.

[3] The composition according to the item [2], wherein the skin wound is a pressure ulcer, a skin ulcer, a burn, a laceration, an abrasion, a leg ulcer, a diabetic ulcer, a venous stasis ulcer, an ischemic (arterial) ulcer, a foot ulcer, a foot gangrene, or a radiation skin ulcer.

[4] The composition according to the item [2], wherein the skin wound is a pressure ulcer.

[5] The composition according to any one of the items [1] to [4], being a preparation for external use.

[6] The composition according to the item [5], wherein the preparation for external use is an ointment, a cream, a foam, a liquid for external use (a lotion or a liniment), a spray, or a hydrogel.

[6-1] The composition according to the item [5], wherein the preparation for external use is an ointment or a cream.

[6-2] The composition according to the item [5], wherein the preparation for external use is an ointment.

[7] The composition according to any one of the items [5], [6], [6-1], and [6-2], wherein the preparation for external use contains N-({2-[4-(difluoromethoxy)-3-(propan-2-yloxy)phenyl]-1,3-oxazol-4-yl}methyl)-2-ethoxybenzamide or its salt in an amount of 0.1 to 10 mass% (for example, (i) 0.1 to 3 mass% or (ii) 0.3 or 1 mass%) in terms of N-({2-[4-(difluoromethoxy)-3-(propan-2-yloxy)phenyl]-1,3-oxazol-4-yl}methyl)-2-ethoxybenzamide.

[8] The composition according to any one of the items [5] to [7], [6-1], and [6-2], characterized in that the composition is administered 1 to 2 times a day.

[9] The composition according to any one of the items [1] to [8], [6-1], and [6-2], being a composition for promotion of epidermis formation.

[10] The composition according to any one of the items [1] to [9], [6-1], and [6-2], characterized in that the composition acts via a fibroblast (preferably, a skin fibroblast).

[11] A composition containing N-({2-[4-(difluoromethoxy)-3-(propan-2-yloxy)phenyl]-1,3-oxazol-4-yl}methyl)-2-ethoxybenzamide or its salt,
the composition for promotion of epidermis formation.

[12] The composition according to the item [11], being a treatment agent for a wound.

[13] The composition according to the item [12], wherein the wound is a skin wound.

[14] The composition according to the item [13], wherein the skin wound is a pressure ulcer, a skin ulcer, a burn, a laceration, an abrasion, a leg ulcer, a diabetic ulcer, a venous stasis ulcer, an ischemic (arterial) ulcer, a foot ulcer, a foot gangrene, or a radiation skin ulcer.

[15] The composition according to the item [13], wherein the skin wound is a pressure ulcer.

[15-1] The composition according to the item [1], wherein the wound is a mucosal wound.

[15-2] The composition according to the item [15-1], wherein the mucosal wound is an oral mucosal wound, an ocular mucosal wound, a nasal mucosal wound, a tracheal mucosal wound, a gastric mucosal wound, a small intestinal mucosal wound, a colorectal mucosal wound, a vesical mucosal wound, a uterine mucosal wound, or a vaginal mucosal wound.

[15-3] The composition according to the item [15-1], wherein the mucosal wound is an oral wound or an ocular mucosal wound.

[15-4] The composition according to the item [15-3], wherein the oral wound is an oral mucositis, a stomatitis, an oral ulcer, an oral erosion, glossitis, or a cheilitis.

[15-5] The composition according to the item [15-3], wherein the ocular mucosal wound is a keratoconjunctival epithelial disorder, a corneal epithelial disorder, a corneal erosion, superficial punctate keratopathy, dry eye, a keratitis accompanied by a corneal wound, or conjunctivitis accompanied by a conjunctival wound.

[15-6] The composition according to the item [15-3], wherein the ocular mucosal wound is dry eye accompanied by a keratoconjunctival epithelial disorder.

[16] The composition according to any one of the items [11] to [15] and [15-1] to [15-6], being a preparation for external use.

[16-1] The composition according to the item [16], wherein the preparation for external use is an eye drop or an eye ointment.

[16-2] The composition according to the item [16], wherein the preparation for external use is a mouthwash, an oral spray, or an oral ointment.

[16-3] The composition according to the item [16-1] or [16-2], wherein the preparation for external use contains N-({2-[4-(difluoromethoxy)-3-(propan-2-yloxy)phenyl]-1,3-oxazol-4-yl}methyl)-2-ethoxybenzamide or its salt in an amount of 0.1 to 10 mass% (for example, (i) 0.1 to 3 mass% or (ii) 0.3 or 1 mass%) in terms of N-({2-[4-(difluoromethoxy)-3-(propan-2-yloxy)phenyl]-1,3-oxazol-4-yl}methyl)-2-ethoxybenzamide.

[16-4] The composition according to any one of the items [16-1] to [16-3], characterized in that the composition is administered 1 to 4 times a day.

[16-5] The composition according to any one of the items [15-1] to [15-4] and [16-1] to [16-4], being a composition for promotion of oral mucosal epithelium formation.

[16-6] The composition according to any one of the items [15-1] to [15-4] and [16-1] to [16-5], characterized in that the composition acts via an oral fibroblast.

[16-7] The composition according to any one of the items [15-1] to [15-3], [15-5] to [15-6], and [16-1] to [16-4], being a composition for promotion of corneal epithelium formation.

[16-8] The composition according to any one of the items [15-1] to [15-3], [15-5] to [15-6], [16-1] to [16-4], and [16-6] to [16-7], characterized in that the composition acts via a keratocyte.

[17] The composition according to the item [16], wherein the preparation for external use is an ointment, a cream, a foam, a liquid for external use (a lotion or a liniment), a spray, or a hydrogel.

[17-1] The composition according to the item [16], wherein the preparation for external use is an ointment or a cream.

[17-2] The composition according to the item [16], wherein the preparation for external use is an ointment.

[18] The composition according to any one of the items [16], [17], [17-1], and [17-2], wherein the preparation for external use contains N-({2-[4-(difluoromethoxy)-3-(propan-2-yloxy)phenyl]-1,3-oxazol-4-yl}methyl)-2-ethoxybenzamide or its salt in an amount of 0.1 to 10 mass% (for example, (i) 0.1 to 3 mass% or (ii) 0.3 or 1 mass%) in terms of N-({2-[4-(difluoromethoxy)-3-(propan-2-yloxy)phenyl]-1,3-oxazol-4-yl}methyl)-2-ethoxybenzamide.

[19] The composition according to any one of the items [16] to [18], [17-1], and [17-2], characterized in that the composition is administered 1 to 2 times a day.

[20] A method of treating a wound, the method including administering an effective dose of N-({2-[4-(difluoromethoxy)-3-(propan-2-yloxy)phenyl]-1,3-oxazol-4-yl}methyl)-2-ethoxybenzamide or its salt to a subject (a human to be treated with the method in an aspect).

[21] Use of N-({2-[4-(difluoromethoxy)-3-(propan-2-yloxy)phenyl]-1,3-oxazol-4-yl}methyl)-2-ethoxybenzamide or its salt,
the use for production of a composition for a treatment for a wound.

[22] N-({2-[4-(difluoromethoxy)-3-(propan-2-yloxy)phenyl]-1,3-oxazol-4-yl}methyl)-2-ethoxybenzamide or its salt for use in a treatment for a wound.

[23] The composition according to any one of the items [1] to [4] and [9] to [15], being a wound dressing.

### Advantageous Effects of Invention

The treatment composition of the present disclosure has exhibited a wound healing effect in a wound model animal. Furthermore, the treatment composition of the present disclosure has exhibited an epidermis formation promoting action. From the above, the compound A or its salt can be used as a wound treatment composition and/or a composition for promotion of epidermis formation.

### Brief Description of Drawings

Fig. 1 shows a change in the area of a defect portion at the time of applying a test substance to a spontaneously diabetic mouse (Mean ± S.E. N = 6)** p < 0.01, N.S. Not Significant, vs vehicle administration group).
Fig. 2 shows a change in a scratch region at the time of adding a test substance or a conditioned medium obtained from a skin fibroblast cultured in a culture medium containing a test substance to epidermic cells having a scratch region (Mean ± S.E., replicate 6-9).
Fig. 3 shows an injury area at the time of administering a test substance to a rat oral mucosal ulcer caused by cauterization (Mean ± S.E. N = 6)* p < 0.05, N.S. Not Significant, vs vehicle administration group).
Fig. 4 shows a healing rate at the time of applying a test substance as eye drops to a rat corneal epithelial defect (Mean ± S.E. N = 5)* p < 0.05, N.S. Not Significant, vs vehicle administration group).
Fig. 5 shows a change in a scratch region at the time of adding a test substance or a conditioned medium obtained from a keratocyte cultured in a culture medium containing a test substance to corneal epithelial cells having a scratch region (Mean ± S.E., replicate 8).

### Description of Embodiments

Hereinafter, each embodiment included in the present disclosure will be described in more detail.

In the present disclosure, N-({2-[4-(difluoromethoxy)-3-(propan-2-yloxy)phenyl]-1,3-oxazol-4-yl}methyl)-2-ethoxybenzamide (a compound A) is a compound having the following structure: 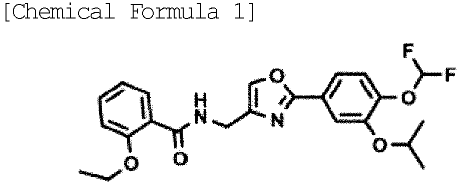 and is also referred to as difamilast. The compound A, its salt, crystals thereof, and preparations containing these can be obtained, for example, in accordance with the methods described in WO2007/058338, JP2008-308496A, WO2014/034958, WO2017/115780, WO2019/194211, and JP2021-59538A.

In the present disclosure, the term "wound" refers to an injury in a tissue covering an outer surface or an inner surface of the body. More specific examples of the wound to be treated by the composition or the agent according to the present disclosure include skin wounds and mucosal wounds (such as oral wounds and ocular mucosal wounds).

In the present disclosure, the term "skin wound" refers to a wound in a skin tissue. The skin tissue is a layer covering the surface of the body, and includes three layers: epidermis, dermis, and a subcutaneous tissue. The epidermis mainly includes four layers (in which the uppermost layer is a corneum). More specific examples of the skin wound to be treated by the composition or the agent according to the present disclosure include pressure ulcers, skin ulcers, burns, lacerations, abrasions, leg ulcers, diabetic ulcers, venous stasis ulcers, ischemic (arterial) ulcers, foot ulcers, foot gangrenes, and radiation skin ulcers. More preferred examples are pressure ulcers and skin ulcers, and still more preferred examples are pressure ulcers.

In the present disclosure, the term "mucosal wound" refers to a wound in a mucosal tissue. The mucosal tissue include a mucosal epithelium as the outermost layer and a connective tissue including a lamina propria and a submucosa under the mucosal epithelium. Examples of the mucosa include oral mucosae, ocular mucosae (corneae and conjunctivae), nasal mucosae, tracheal mucosae, gastric mucosae, intestinal mucosae, vesical mucosae, uterine mucosae, and genital mucosae. Examples of the mucosal wound to be treated by the composition or the agent according to the present disclosure include oral mucosal wounds, ocular mucosal wounds (corneal wounds and conjunctival wounds), nasal mucosal wounds, tracheal mucosal wounds, gastric mucosal wounds, small intestinal mucosal wounds, colorectal mucosal wounds, vesical mucosal wounds, uterine mucosal wounds, and vaginal mucosal wounds, and more preferred examples are oral mucosal wounds and ocular mucosal wounds.

In the present disclosure, the term "oral mucosal wound" refers to a wound in an oral mucosal tissue. Examples of the oral mucosal wound to be treated by the composition or the agent according to the present disclosure include oral mucositides (stomatitides), oral ulcers, oral erosions, glossitis, and cheilitides, and more specific examples of the oral mucosal wound include aphthous stomatitides, aphthous oral ulcers, viral stomatitides, mycotic stomatitides, stomatitides associated with cancer treatment (for example, an anticancer agent or radiotherapy), and stomatitides associated with Sjogren's syndrome or Stevens-Johnson syndrome. More preferred examples are aphthous stomatitides and stomatitides associated with cancer treatment, and still more preferred examples are stomatitides induced by an anticancer agent or radiotherapy.

In the present disclosure, the term "ocular mucosal wound" refers to a wound in an ocular mucosal tissue, and examples of the ocular mucosal wound include keratoconjunctival epithelial disorders. The term "keratoconjunctival epithelial disorder" refers to a disorder in a corneal epithelial tissue and/or a conjunctival epithelial tissue. More specific examples of the ocular mucosal wound or the keratoconjunctival epithelial disorder to be treated by the composition or the agent according to the present disclosure include corneal epithelial disorders, conjunctival epithelial disorders, corneal erosions, superficial punctate keratopathy, dry eye, keratitides accompanied by a corneal wound, and conjunctivitis accompanied by a conjunctival wound. More preferred examples are keratoconjunctival epithelial disorders, corneal erosions, superficial punctate keratopathy, and dry eye, and still more preferred examples are keratoconjunctival epithelial disorders, superficial punctate keratopathy, and dry eye accompanied by a keratoconjunctival epithelial disorder.

In the present disclosure, a method of evaluating a treatment effect on a wound is not particularly limited as long as it is a system that can evaluate a treatment effect on a wound. The evaluation can be performed using, for example, a change in a wound portion area as an index.

In the present disclosure, the term "promotion of epidermis formation" refers to repairing an injured epidermis or promoting repair of an injured epidermis in an aspect. A method of evaluating the promotion of epidermis formation is not particularly limited as long as it is a system that can evaluate an epidermis formation promoting action. The evaluation can be performed using, for example, a change in a scratch region of an epidermic cell culture medium as an index. In a method of evaluating whether the epidermis formation promoting action is performed via a fibroblast, a conditioned medium is collected from skin fibroblasts cultured in a culture medium containing an evaluation substance, then the conditioned medium is added to epidermic cells having a scratch region, and a change in the scratch region and the like are confirmed to perform the evaluation.

In the present disclosure, the term "promotion of oral mucosal epithelium formation" refers to repairing an injured oral mucosal epithelium or promoting repair of an injured oral mucosal epithelium in an aspect. A method of evaluating the promotion of oral mucosal epithelium formation is not particularly limited as long as it is a system that can evaluate an oral mucosal epithelium formation promoting action. The evaluation can be performed using, for example, a change in a scratch region of oral mucosal epithelial cells as an index. In a method of evaluating whether the oral mucosal epithelium formation promoting action is performed via an oral fibroblast, a conditioned medium is collected from oral fibroblasts cultured in a culture medium containing an evaluation substance, then the conditioned medium is added to oral mucosal epithelial cells having a scratch region, and a change in the scratch region and the like are confirmed to perform the evaluation.

In the present disclosure, the term "promotion of corneal epithelium formation" refers to repairing an injured corneal epithelium or promoting repair of an injured corneal epithelium in an aspect. A method of evaluating the promotion of corneal epithelium formation is not particularly limited as long as it is a system that can evaluate a corneal epithelium formation promoting action. The evaluation can be performed using, for example, a change in a scratch region of corneal epithelial cells as an index. In a method of evaluating whether the corneal epithelium formation promoting action is performed via a keratocyte, a conditioned medium is collected from keratocytes cultured in a culture medium containing an evaluation substance, then the conditioned medium is added to corneal epithelial cells having a scratch region, and a change in the scratch region and the like are confirmed to perform the evaluation.

### Salt

The compound A can be converted into a salt with a known method.

In the present disclosure, the salt of the compound A is, for example, a pharmaceutically acceptable salt.

In the present disclosure, examples of the salt of the compound A include acid addition salts, alkali metal salts, alkaline earth metal salts, ammonium salts, and amine salts.

Examples of the acid addition salts among the above include inorganic acid salts such as hydrochlorides, hydrobromides, hydroiodides, sulfates, phosphates, and nitrates and organic acid salts such as acetates, lactates, tartrates, benzoates, citrates, methanesulfonates, ethanesulfonates, trifluoroacetates, benzenesulfonates, toluenesulfonates, isethionates, glucuronates, and gluconates.

Examples of the alkali metal salts include sodium salts and potassium salts.

Examples of the alkaline earth metal salts include calcium salts and magnesium salts.

Examples of the ammonium salts include tetramethylammonium salts.

Examples of the amine salts include triethylamine salts, methylamine salts, dimethylamine salts, cyclopentylamine salts, benzylamine salts, phenethylamine salts, piperidine salts, monoethanolamine salts, diethanolamine salts, tris(hydroxymethyl)aminomethane salts, lysine salts, arginine salts, and N-methyl-D-glucamine salts.

### Solvate/cocrystal

The compound A or its salt may exist in the form of a solvate or a cocrystal, and a compound existing in such a form is also included in the compound A or its salt.

A solvate of the compound A or its salt can be prepared with a known method. Examples of the solvate include solvates of hydrates and alcohol-based solvents (such as ethanol).

A cocrystal of the compound A can be prepared with a known method. The cocrystal is preferably pharmaceutically acceptable. A cocrystal is defined as a crystal formed by an intermolecular interaction different from an ionic bond between two or more different molecules. Examples of a suitable cocrystal former include those described in WO2006/007448 including 4-aminobenzoic acid, 4-aminopyridine, adenine, alanine, and acetylsalicylic acid.

### Prodrug

The compound A or its salt can be converted into a prodrug with a known method. The prodrug is preferably pharmaceutically acceptable, and examples of the prodrug include those described in Prog. Med., 5, 2157-2161 (1985) and "Pharmaceutical research and development" (published by Hirokawa Shoten Co., 1990), Volume 7, Molecular Design 163-198.

The compound A or its salt also includes a compound obtained by converting a constituent atom into an isotope of the atom (for example, ²H, ³H, ¹³C, and ¹⁴C) .

The compound A or its salt may be a crystal polymorph, and the crystal polymorph is also included in the compound A or its salt.

### Toxicity

The compound A of the present disclosure or its salt, or a composition or an agent containing the compound A or its salt has sufficiently low toxicity, and can be safely used as a medicine.

### Preparation

Examples of the composition or the agent of the present disclosure include preparations for external use, tablets, capsules, granules, powders, syrups, suspensions, injections, suppositories, eye drops, ointments (such as eye ointments and oral ointments), oral liquids, mouthwashes, oral sprays, nasal drops, enemas, creams, foams, liquids for external use (lotions and liniments), sprays, hydrogels, coatings, patches, inhalants, and wound dressings, and these can be prepared with known methods. Particularly preferred examples are preparations for external use.

Examples of an aspect of the preparation for external use according to the present disclosure include ointments, creams, foams, liquids for external use (lotions and liniments), sprays, hydrogels, eye drops, eye ointments, mouthwashes, oral sprays, oral ointments, and oral patches, and preferred examples among them are ointments (particularly, eye ointments and oral ointments), creams, eye drops, mouthwashes, and oral sprays.

Examples of aspects of the ointments (particularly, eye ointments and oral ointments) include water-soluble ointments and oleaginous ointments. Examples of an aspect of the cream include water-in-oil creams and oil-in-water creams.

In a case where the ointment according to the present disclosure is an oleaginous ointment, the oleaginous ointment preferably contains, for example, the compound A, a solvent that dissolves the compound A, and an ointment base that disperses or dissolves the solvent. Examples of the solvent include ethylene carbonate, propylene carbonate, benzyl alcohol, and triacetin. These can be used singly or in combination of two or more kinds thereof. Examples of the ointment base include petrolatum, paraffin, wax, and beeswax. These can be used singly or in combination of two or more kinds thereof. More specifically, the oleaginous ointment is, for example, an ointment containing the compound A, white petrolatum, liquid paraffin, white beeswax, paraffin, and propylene carbonate.

The compound A or its salt can be used in a wound dressing to be used for promotion of skin wound healing. Specifically, the compound A or its salt is contained in a substrate for covering of a wound, and thus can be provided as a wound dressing. This wound dressing can appropriately and uniformly provide an effect of the present invention for the entire wound site. Furthermore, the wound dressing can be used according to the size of a wound, and thus an effect of the present invention can be efficiently provided.

In the present disclosure, the term "wound dressing" means a material that is attached so as to cover a wound site and thus used for promoting wound healing. Here, the term "wound site" means an injured site on the body surface, such as a pressure ulcer, a skin ulcer, a burn, a laceration, an abrasion, a leg ulcer, a diabetic ulcer, a venous stasis ulcer, an ischemic (arterial) ulcer, a foot ulcer, a foot gangrene, or a radiation skin ulcer. Examples of the wound dressing also include dressings to be used for the purpose of creating a moist environment in a wound.

The wound dressing can include, for example, a composition (for example, a preparation for external use of the present disclosure) including a substrate for covering of a wound site and the compound A of the present disclosure or its salt contained in the substrate with a method such as impregnation or application. Examples of the substrate of the wound dressing include adhesive bandages, adhesive tapes, polyurethane films, gauze, hydrogels, alginates, hydrofibers, and hydropolymer hydrocolloids. Examples of the method of making a substrate contain the compound A or its salt include a method of applying the composition to a substrate and a method of mixing the composition with a raw material included in a substrate.

The composition or the agent of the present disclosure may include a pharmaceutically acceptable carrier. The composition or the agent of the present disclosure can be prepared by combining the compound A or its salt with, for example, a pharmaceutically acceptable carrier or the like. The preparation can be carried out with a known method or a method that can be easily conceived from a known method.

Methods of administering the composition or the agent according to the present disclosure include peroral administration and parenteral (for example, venous, arterial, muscular, subcutaneous, peritoneal, rectal, cutaneous, and topical) administration. In a case where the composition or the agent according to the present disclosure is a preparation for external use, examples of the method of administering the composition or the agent according to the present disclosure include methods of application, spray, coating, and attachment.

The composition or the agent according to the present disclosure contains, for example, 0.01 to 10 mass% of the compound A or its salt in terms of the compound A. The upper limit or the lower limit of the range may be, for example, 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, or 9.5 mass%. For example, the range may be 0.1 to 3 mass%, 0.3 to 1 mass%, 0.3 mass%, or 1 mass%.

The composition or the agent according to the present disclosure is administered, for example, 1 to 6 times a day, 1 to 4 times a day, 1 to 3 times a day, 1 to 2 times a day, or 2 times a day.

In a case where the composition or the agent according to the present disclosure is a preparation for external use (for example, an ointment), the preparation for external use of the present disclosure can be applied, for an adult, to an affected part in the form of a 1 mass% preparation in an appropriate amount twice a day, and in another aspect for a child, the composition or the agent according to the present disclosure can be applied to an affected part in the form of a 0.3 mass% preparation in an appropriate amount twice a day, or according to the symptom, in the form of a 1 mass% preparation in an appropriate amount twice a day. The preparation for external use of the present disclosure after being applied to an affected part can be covered with a dressing or the like.

In a case where the composition or the agent according to the present disclosure is a wound dressing, examples of a method of using the composition or the agent include a method in which a base (for example, a polyurethane film) to which the compound A or its salt is applied in advance is attached to a wound portion, and a method in which a base (for example, hydrogel) in which the compound A or its salt is mixed in advance is used at a wound portion and further covered with another base (polyurethane film or the like). The wound dressing may further include another base (for example, an elastic adhesive bandage), for example, in order to fix these bases.

In a case where the composition or the agent of the present disclosure is orally administered, the daily dosage is, for example, about 0.0001 to 100 mg/kg, 0.01 to 30 mg/kg, or 0.03 to 10 mg/kg per body weight, and the composition or the agent can be administered once to a plurality of times a day. In a case where the composition or the agent of the present disclosure is administered intravenously, the daily dosage is, for example, about 0.00001 to 10 mg/kg per body weight, and the composition or the agent can be administered once to a plurality of times a day. As the topical agent, for example, the composition or the agent can be administered at a dosage of about 0.0001 to 100 mg/kg body weight once to a plurality of times a day.

### Combination/compounding agent

The compound A according to the present disclosure or its salt may be used in combination with another drug to be used for wound treatment. In the present disclosure, the administration form in the case of use in combination with another drug (combination) may be a form of a compounding agent obtained by compounding a plurality of ingredients into one preparation, or a form of administering separate preparations. The combination can complement the effects of another drug for prevention, suppression of symptom progression, suppression of recurrence, and/or treatment, and can maintain or propose the dosage or the times of administration. In a case where the compound A of the present disclosure or its salt and another drug are separately administered, they may be simultaneously administered for a certain period of time, and then only the compound A or its salt, or only another drug may be administered. Alternatively, the compound A according to the present disclosure or its salt may be administered first and another drug may be administered after completion of the administration, or another drug may be administered first and the compound A according to the present disclosure or its salt may be administered after completion of the administration.

Examples of another drug to be used in combination with the compound A according to the present disclosure or its salt include prostaglandin E1 preparations (for example, alprostadil alfadex (PROSTANDIN (trademark) ointment)), basic fibroblast growth factor (bFGF) preparations (trafermin (FIBROBLAST (trademark) spray, and the like), cyclic derivatives (bucladesine sodium (Actosin (trademark) ointment, and the like), sucrose/ povidone-iodine (U-PASTA KOWA ointment, ISODINE (trademark) sugar paste ointment), povidone-iodine (ISODINE (trademark) gel 10%), bromelain (bromelain ointment), white petrolatum (PROPETO (trademark)), zinc oxide (zinc oxide ointment), dimethyl isopropylazulene (Azunol (trademark) ointment), sulfadiazine silver (GEBEN (trademark) cream), and lysozyme hydrochloride (REFLAP (trademark) ointment).

In the present disclosure, the term "treatment" refers to healing or ameliorating a disease or a symptom of the disease, and includes prevention or suppression of symptom progression in an aspect. In the present disclosure, the term "prevention" means preventing the onset of a disease or a symptom or retarding the onset for a certain period of time, and includes suppression of recurrence in an aspect. In the present disclosure, the term "suppression of recurrence" means preventing recurrence of a disease or a symptom or reducing the possibility of the recurrence. In the present disclosure, the term "suppression of symptom progression" means suppressing progression or aggravation of a symptom to stop progression of the disease state.

The present disclosure also includes a composition for promotion of epidermis formation containing N-({2-[4-(difluoromethoxy)-3-(propan-2-yloxy)phenyl]-1,3-oxazol-4-yl}methyl)-2-ethoxybenzamide or its salt. For the "composition for promotion of epidermis formation", the above description of the "composition according to the present disclosure" can be incorporated.

The present disclosure also includes a composition for promotion of oral mucosal epithelium formation containing N-({2-[4-(difluoromethoxy)-3-(propan-2-yloxy)phenyl]-1,3-oxazol-4-yl}methyl)-2-ethoxybenzamide or its salt. For the "composition for promotion of oral mucosal epithelium formation", the above description of the "composition according to the present disclosure" can be incorporated.

The present disclosure also includes a composition for promotion of corneal epithelium formation containing N-({2-[4-(difluoromethoxy)-3-(propan-2-yloxy)phenyl]-1,3-oxazol-4-yl}methyl)-2-ethoxybenzamide or its salt. For the "composition for promotion of corneal epithelium formation", the above description of the "composition according to the present disclosure" can be incorporated.

The present disclosure also includes a method of treating a wound including administering an effective dose of N-({2-[4-(difluoromethoxy)-3-(propan-2-yloxy)phenyl]-1,3-oxazol-4-yl}methyl)-2-ethoxybenzamide or its salt to a subject. For the "method of treating a wound", the above description of the "composition according to the present disclosure" can be incorporated.

The subject of administration is not particularly limited as long as the subject needs a treatment of a wound.

In the present description, the term "comprising" or "including" includes "consisting essentially of" and "consisting of."

Various characteristics (properties, structures, functions, and the like) described in each embodiment described above may be combined in any manner in specifying the subject matter included in the present disclosure.

### Examples

Hereinafter, the subject matter included in the present disclosure will be described more specifically, but the subject matter is not limited to the following examples. Unless otherwise specified, "%" means "% by mass".

### Example 1. influence of compound A (N-({2-[4-(difluoromethoxy)-3-(propan-2-yloxy)phenyl]-1,3-oxazol-4-yl}methyl)-2-ethoxybenzamide) on wound model using diabetic mouse

The compound A or crisaborole was administered to db/db mice, which are spontaneously diabetic models, for 10 days, and the influence on wound healing was examined by analyzing the wound area.

### Test method

Before start of the test, the db/db mice were grouped using the HbAlc blood concentration as an index, and shaved from the back to the abdomen. On the test start day, the back skin was punched using a biopsy punch (ϕ6mm) under isoflurane anesthesia, and thus two circular defect wounds were prepared. The defect portions were photographed, and then an ointment preparation (vehicle [30% medium chain fatty acid triglyceride-containing white petrolatum], compound A or crisaborole) as a test substance was applied to the defect portions and covered with a dressing (Tegaderm, 3M). After the day following the preparation of the defect wounds, removal of the dressing, photographing of the defect portions (once every two days), administration of the test substance, and covering with a dressing were repeated (once daily for 10 days).

### Results

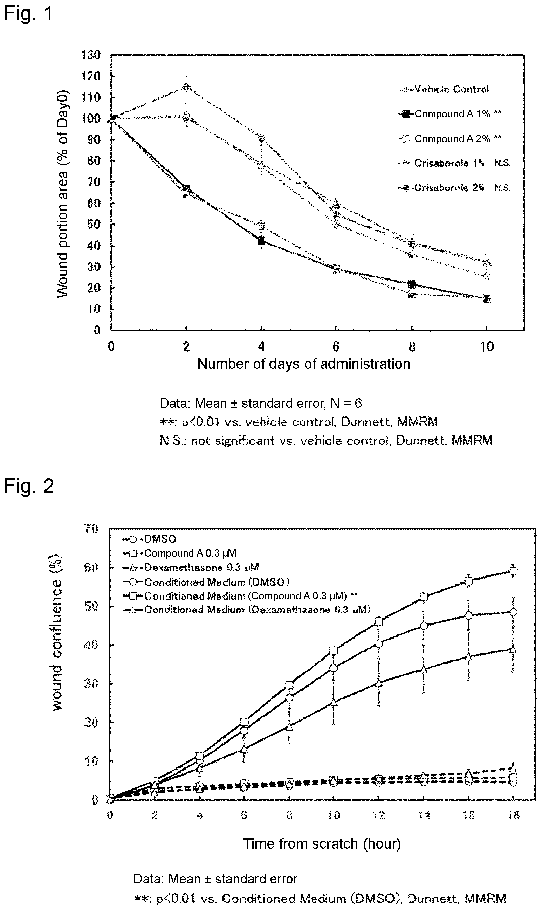

Fig. 1 shows the results.

In the vehicle administration group, the area of the defect wounds gradually decreased from the fourth day and thus start of healing was confirmed, and in the compound A groups (1%, 2%), the area of the defect portions rapidly decreased from the second day and thus start of healing was confirmed, and a healing promoting action was confirmed over the experimental period as compared with the vehicle administration group (p < 0.01) .

In the case of crisaborole, which is also a PDE4 inhibitor, it was confirmed that the area of the defect portions increased on the second day (in the 2% group) and then gradually decreased from the fourth day, and thus a significant healing promoting action was not observed as compared with the vehicle administration group.

### Example 2. influence of compound A on epidermic cells via skin fibroblast

The influence of the compound A on epidermic cells via a skin fibroblast was evaluated. As the epidermic cell and the skin fibroblast used in the evaluation system, a normal human epidermal keratinocyte (NHEK, Kurabo Industries Ltd.) and a normal human dermal fibroblast (NHDF, Kurabo Industries Ltd.) were used. As a reference compound, dexamethasone, which is an anti-inflammatory drug, was used.

### Test method

Skin fibroblasts (1 × 10⁵ cells/mL) suspended in Dulbecco's Modified Eagle Medium (DMEM) containing 10% (v/v) fetal bovine serum (FBS) were seeded at 1 mL/well in a 12 well plate coated with type I collagen, and cultured for 48 hours. After removal of the culture medium and washing with phosphate buffered saline (PBS), the compound solution was added. As the compound solution, a solution was used that was prepared by mixing dimethyl sulfoxide or a compound (the compound A or dexamethasone) dissolved in dimethyl sulfoxide with a normal human epidermal keratinocyte basal medium (HuMedia-KB2, Kurabo Industries Ltd.) (the final concentration of dimethyl sulfoxide was 0.1% [v/v]). After completion of the culture for 24 hours, the conditioned medium was collected.

Epidermic cells (6 × 10⁵ cells/mL) suspended in HuMedia-KB2 were seeded at 100 µL/well in ImageLock 96-well Plate (Sartorius AG) coated with type I collagen, and cultured for 4 hours. Each well was scratched using a Wound Maker (trademark) tool (Sartorius AG) to prepare an initial scratch wound area in which no cells were present. Each well was washed with HuMedia-KB2, then the compound solution or the conditioned medium was added to the well, and culture was performed for 18 hours. During the culture, images were acquired over time with Incucyte (trademark) S3 (Sartorius AG). Image analysis was performed using Scratch Wound Cell Migration Software Module (Sartorius AG) to calculate the percentage of the area covered with cells in the initial scratch wound area (wound confluence [%]).

### Results

Fig. 2 shows the results.

The addition of the conditioned medium from the skin fibroblasts induced migration of the epidermic cells. The compound A promoted migration of the epidermic cells via the skin fibroblasts, and thus an epidermis formation promoting action of the compound A has been suggested.

### Example 3. influence of compound A on wound model using oral ulcer rat

### Test method

Using a male rat (SD rat), an oral ulcer was initiated by cauterization as follows. That is, conventional rats were anesthetized by isoflurane inhalation anesthesia and then placed on their back, and while the upper jaw and the lower jaw of each rat were opened using a rib retractor to take a view, a monopolar having a tip diameter of 2 mm was applied to the central portion of the left oral mucosa, and the portion was cauterized in a circular shape (diameter: 3 to 4 mm) for about 10 to 20 seconds at a set output of 20 to induce an oral ulcer. After the cauterization, the rat was returned to a breeding cage and naturally awakened.

The day of oral ulcer induction was defined as the start day (Day 0). Two days after the oral ulcer induction (Day 2), the rats were grouped using a stratified random sampling method based on the body weight. From 3 days after the initiation of an oral ulcer by cauterization (Day 3), an ointment preparation containing 1 mass% of a test substance or an ointment base alone was orally administered at a dose of 0.05 mL twice a day (around 8:00 and 16:00) for 5 days. (As the compound A 1%, Moizerto (registered trademark) ointment 1% was used. The crisaborole 1% was prepared using a formula in which Moizerto (registered trademark) ointment 1% as the compound A was replaced by crisaborole.) Specifically, a rat anesthetized by isoflurane inhalation was laid down in a left side position, and the ointment preparation was administered to the left cheek where an ulcer was prepared in a state where the oral cavity was opened using tweezers or a rib retractor.

### Results

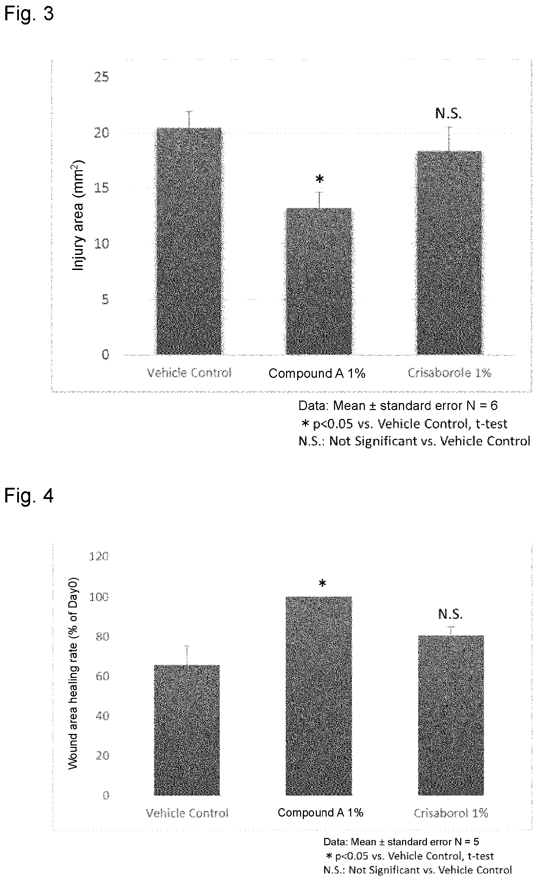

Fig. 3 shows the results of the oral ulcer area on Day 8.

In the compound A administration group, significant reduction in the oral ulcer area was observed as compared with the vehicle administration group (vehicle control) (n = 6, p < 0.05, t-test). The ulcer area in the vehicle administration group was 20.4 ± 2.3 mm², whereas the ulcer area in the compound A administration group was 13.1 ± 1.3 mm².

Meanwhile, in the crisaborole administration group, significant reduction in the oral ulcer area was not observed as compared with the vehicle administration group (n = 6, n.s., t-test). The ulcer area in the crisaborole administration group was 18.3 ± 2.7 mm².

### Example 4. influence of compound A on wound model using corneal wound rat

### Test method

Using a male rat (SD rat), a corneal wound was induced as follows. On the day before the start of the test, rats were grouped using a stratified random sampling method based on the body weight. Under isoflurane anesthesia, each conventional rat was anesthetized with a Benoxil ophthalmic solution to the surface of the eye, and then a nonwoven fabric (3 mm in diameter) with 1-heptanol immersed was placed on the central portion of the cornea and allowed to stand for 30 seconds. Thereafter, the surface of the eye was washed with physiological saline, and the corneal epithelium was scratched with a cotton swab and thus carefully peeled to prepare a corneal defect. A 0.5% methylene blue solution was instilled into the eye, and the corneal epithelial defect portion was washed with physiological saline and then photographed, and then an ointment preparation (base alone, 1% compound A-containing ointment, or 1% crisaborole-containing ointment) as a test substance was administered to the eye four times a day (around 8:00, 11:00, 14:00, and 17:00) for three days. After the day following the preparation of the defect wound, photographing of the defect portion and administration of the test substance were repeated.

### Results

Fig. 4 shows the results of the wound area healing rate on Day 2.

In the vehicle administration group (vehicle control), a gradual decrease in the defect wound area was confirmed, and apparent healing started on Day 2. Meanwhile, in the compound A administration group (1%), healing started earlier than in the vehicle administration group, the defect portion area rapidly decreased from 8 hours after preparation of the defect wound, a significant healing promoting action was confirmed as compared with the vehicle administration group (n = 5, p < 0.05, t-test), and complete healing (healing rate: 100%) was confirmed on Day 2. Meanwhile, in the crisaborole administration group (1%), a defect wound was also observed on Day 2, and healing was not completed as compared with the vehicle administration group (healing rate: 81.6%) (n = 5, n.s., t-test).

### Example 5. influence of compound A on corneal epithelial cells via keratocyte

The influence of the compound A on corneal epithelial cells via a keratocyte was evaluated. As the corneal epithelial cell and the keratocyte used in the evaluation system, a normal human corneal epithelial cell (HCEC-2, Kurabo Industries Ltd.) and a normal human keratocyte (HK, Cosmo Bio Co., Ltd.) were used.

### Test method

Keratocytes (2 × 10⁵ cells/mL) suspended in a fibroblast culture medium (Fibroblast Medium, Cosmo Bio Co., Ltd.) were seeded at 1 mL/well in a 12 well plate coated with poly-L-lysine, and cultured overnight. After removal of the culture medium and washing with normal human corneal epithelial cell basal medium (OcuLife BM, Kurabo Industries Ltd.), the compound solution was added. As the compound solution, a solution was used that was prepared by mixing dimethyl sulfoxide or the compound A dissolved in dimethyl sulfoxide with OcuLife BM (the final concentration of dimethyl sulfoxide was 0.1% [v/v]). After completion of the culture for 24 hours, the conditioned medium was collected.

Corneal epithelial cells (6 × 10⁵ cells/mL) suspended in OcuLife BM were seeded at 100 µL/well in ImageLock 96-well Plate (Sartorius AG) coated with type I collagen, and cultured for 4 hours. Each well was scratched using a Wound Maker (trademark) tool (Sartorius AG) to prepare an initial scratch wound area in which no cells were present. Each well was washed with OcuLife BM, then the compound solution or the conditioned medium was added to the well, and culture was performed for 20 hours. During the culture, images were acquired over time with Incucyte (trademark) SX5 (Sartorius AG). Image analysis was performed using Scratch Wound Cell Migration Software Module (Sartorius AG) to calculate the percentage of the area covered with cells in the initial scratch wound area (wound confluence [%]).

### Results

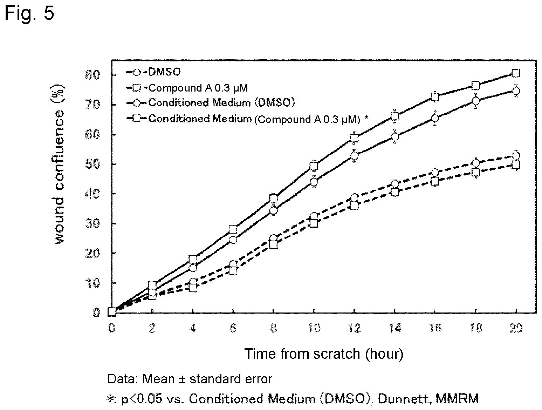

Fig. 5 shows the results.

The addition of the conditioned medium from the keratocytes induced migration of the corneal epithelial cells. The compound A promoted migration of the corneal epithelial cells via the keratocytes, and thus promotion of corneal epithelium formation by the compound A has been suggested.

### Preparation examples

### Preparation example 1

The following ingredients are mixed with a conventional method, and then compressed to obtain a tablet containing 10 mg of the active ingredient in one tablet.
· N-({2-[4-(difluoromethoxy)-3-(propan-2-yloxy)phenyl]-1,3-oxazol-4-yl}methyl)-2-ethoxybenzamide: 10 g
· Mannitol: 168 g
· Hydroxypropyl cellulose: 20 g
· Magnesium stearate: 2 g

### Preparation example 2

The following ingredients are mixed with a conventional method to obtain a preparation for external use containing 1% of the active ingredient in the preparation.
· N-({2-[4-(difluoromethoxy)-3-(propan-2-yloxy)phenyl]-1,3-oxazol-4-yl}methyl)-2-ethoxybenzamide: 10 g
· Petrolatum: 990 g

### Industrial Applicability

N-({2-[4-(difluoromethoxy)-3-(propan-2-yloxy)phenyl]-1,3-oxazol-4-yl}methyl)-2-ethoxybenzamide or its salt is useful as an active ingredient of a composition for a treatment for a wound (such as a pressure ulcer).

## Claims

1. A composition comprising N-({2-[4-(difluoromethoxy)-3-(propan-2-yloxy)phenyl]-1,3-oxazol-4-yl}methyl)-2-ethoxybenzamide or its salt,
the composition for a treatment for a wound.

2. The composition according to claim 1, wherein the wound is a skin wound.

3. The composition according to claim 2, wherein the skin wound is a pressure ulcer, a skin ulcer, a burn, a laceration, an abrasion, a leg ulcer, a diabetic ulcer, a venous stasis ulcer, an ischemic (arterial) ulcer, a foot ulcer, a foot gangrene, or a radiation skin ulcer.

4. The composition according to claim 2, wherein the skin wound is a pressure ulcer.

5. The composition according to any one of claims 1 to 4, being a composition for promotion of epidermis formation.

6. The composition according to any one of claims 1 to 5, **characterized in that** the composition acts via a fibroblast.

7. The composition according to claim 1, wherein the wound is a mucosal wound.

8. The composition according to claim 7, wherein the mucosal wound is an oral mucosal wound or an ocular mucosal wound.

9. The composition according to claim 8, wherein
the mucosal wound is an oral mucosal wound, and
the oral mucosal wound is an oral mucositis, a stomatitis, an oral ulcer, an oral erosion, a glossitis, or a cheilitis.

10. The composition according to claim 8, wherein
the mucosal wound is an ocular mucosal wound, and
the ocular mucosal wound is a keratoconjunctival epithelial disorder, a corneal epithelial disorder, a corneal erosion, superficial punctate keratopathy, dry eye, a keratitis accompanied by a corneal wound, or conjunctivitis accompanied by a conjunctival wound.

11. The composition according to claim 8, wherein
the mucosal wound is an ocular mucosal wound, and
the ocular mucosal wound is dry eye accompanied by a keratoconjunctival epithelial disorder.

12. The composition according to any one of claims 1 to 11, being a preparation for external use.

13. The composition according to claim 12, wherein the preparation for external use is an ointment or a cream.

14. The composition according to claim 12, wherein the preparation for external use is an eye drop or an eye ointment.

15. The composition according to claim 12, wherein the preparation for external use is a mouthwash, an oral spray, or an oral ointment.

16. The composition according to any one of claims 12 to 15, wherein the preparation for external use contains N-({2-[4-(difluoromethoxy)-3-(propan-2-yloxy)phenyl]-1,3-oxazol-4-yl}methyl)-2-ethoxybenzamide or its salt in an amount of 0.1 to 10 mass% in terms of N-({2-[4-(difluoromethoxy)-3-(propan-2-yloxy)phenyl]-1,3-oxazol-4-yl}methyl)-2-ethoxybenzamide.

17. The composition according to any one of claims 12 to 16, **characterized in that** the composition is administered 1 to 2 times a day.

18. The composition according to any one of claims 12 to 16, **characterized in that** the composition is administered 1 to 4 times a day.

19. The composition according to any one of claims 1 to 6, being a wound dressing.
